# EUROPEAN PATENT SPECIFICATION

(11) **EP 2 139 488 B1**
(45) Date of publication and mention of the grant of the patent: **15.08.2012**
(21) Application number: 08715988.5
(22) Date of filing: 22.02.2008
(51) Int. Cl.: A61K 31/525, A61P 9/12

(54) **USE OF RIBOFLAVIN IN THE TREATMENT OF HYPERTENSION**
VERWENDUNG VONR RIBOFLAVIN ZUR BEHANDLUNG VON HYPERTONIE
UTILISATION DE LA RIBOFLAVINE POUR LE TRAITEMENT DE L'HYPERTENSION

(30) Priority: 23.02.2007 GB 0703514
(43) Date of publication of application: 06.01.2010
(73) Proprietor: University Of Ulster, County Londonderry BT52 1SA (GB); The Provost, Fellows and Scholars of the Holy and Undivided Trinity of Queen Elizabeth, near Dublin, Dublin 2 (IE)
(72) Inventor: WARD, Mary, County Londonderry BT55 7FG (GB); MCNULTY, Helene, County Londonderry BT55 7BQ (GB); HORIGAN, Geraldine, County Londonderry BT47 3XN (GB); STRAIN, Sean, County Londonderry BT55 7LN (GB); SCOTT, John, Dublin 14 (IE); PURVIS, John, County Londonderry BT49 0TP (GB)
(74) Representative: Jennings, Tara Romaine
(86) International application number: PCT/EP2008/001437
(87) International publication number: WO 2008/101724

(56) References cited:
- WO-A-80/02799
- FROSST P ET AL: "A CANDIDATE GENETIC RISK FACTOR FOR VASCULAR DISEASE: A COMMON MUTATION IN METHYLENETETRAHYDROFOLATE REDUCTASE" NATURE GENETICS, NEW YORK, NY, US, vol. 10, no. 1, May 1995 (1995-05), pages 111-113, XP000198175 ISSN: 1061-4036 cited in the application
- MCNULTY HELENE ET AL: "Riboflavin lowers homocysteine in individuals homozygous for the MTHFR 677C -> T polymorphism" CIRCULATION, AMERICAN HEART ASSOCIATION, DALLAS, TX, US, vol. 113, no. 1, 3 January 2006 (2006-01-03), pages 74-80, XP009098142 ISSN: 0009-7322
- MUDA PIIBE ET AL: "Homocysteine and red blood cell glutathione as indices for middle-aged untreated essential hypertension patients" JOURNAL OF HYPERTENSION, vol. 21, no. 12, December 2003 (2003-12), pages 2329-2333, XP009098148 ISSN: 0263-6352
- HUSTAD STEINAR ET AL: "Riboflavin as a determinant of plasma total homocysteine: Effect modification by the methylenetetrahydrofolate reductase C677T polymorphism" CLINICAL CHEMISTRY, vol. 46, no. 8 Part 1, August 2000 (2000-08), pages 1065-1071, XP002475249 ISSN: 0009-9147
- UELAND P M ET AL: "Biological and clinical implications of the MTHFR C677T polymorphism" TRENDS IN PHARMACOLOGICAL SCIENCES, ELSEVIER, HAYWARTH, GB, vol. 22, no. 4, 1 April 2001 (2001-04-01), pages 195-201, XP004231954 ISSN: 0165-6147

## Description

The present invention relates to the use of riboflavin in the treatment of hypertension in a genotype specific population.

Hypertension, commonly referred to as high blood pressure, is a medical condition where the blood pressure is elevated, generally chronically elevated. Hypertension of any aetiology is one of the major risk factors for cardiovascular disease (CVD), which includes heart disease and stroke.

It has been found that there is an increased risk of CVD in individuals homozygous for the commonly occurring C677T polymorphism in the gene coding for the enzyme 5, 10-methylenetetrahydrofolate reductase (MTHFR). MTHFR is required for the formation of 5-methyltetrahydrofolate which in turn is required to convert homocysteine to methionine. Individuals who are homozygous for the MTHFR C677T polymorphism (TT genotype) are considered to have a significantly higher risk of heart disease and stroke than those with the wild-type gene (CC genotype). People who are heterozygous for the polymorphism (CT genotype) may also have a moderately higher risk of heart disease and stroke. Various anti-hypertensive drugs (e.g. ACE inhibitors, beta blockers and diuretics) are available to lower blood pressure in those deemed clinically to have hypertension. However, these drugs can have undesirable side effects and some subjects may remain hypertensive despite being tried on more than one type of anti-hypertensive drug.

Pharmaceutical compositions comprising riboflavin are disclosed in US2007031394 and in WO0019817.

The effect of riboflavin in lowering homocystein levels in individuals homozygous for the MTHFR 677C -> T Polymorphism is disclosed in McNulty et al., 2006, Circulation American Heart Association, Dallas, Texas, US, vol. 113(1):74-80.

WO80/02799 discloses riboflavin analogues as antihypertensive agents.

It has now surprisingly been found that riboflavin (vitamin B2) has a significant systolic and diastolic blood pressure lowering effect, specifically in CVD patients homozygous for the MTHFR C677T polymorphism.

According to a first aspect of the present invention, there is provided the use of riboflavin in the manufacture of a medicament for the treatment or prophylaxis of elevated blood pressure in a subject homozygous or heterozygous for the MTHFR C677T polymorphism.

According to a second aspect of the present invention, there is provided a pharmaceutical product for the treatment or prophylaxis of elevated blood pressure in a subject homozygous or heterozygous for the MTHFR C677T polymorphism, comprising a pharmaceutically effective amount of an anti-hypertensive agent and riboflavin, for simultaneous, separate or sequential administration.

Suitable anti-hypertensive agents include ACE inhibitors such as quinapril, captopril, lisinopril, benazepril, perindopril, enalapril maleate, trandolapril, ramipril and cilazapril; beta blockers such as atenolol, tertatolol, metoprolol tartrate, bisoprolol fumarate, nebbivolol, celiprolol and pindolol; Ca⁺⁺ antagonists such as nifedipine, diltiazem, amlodipine, verapamil and felopidine; alpha blockers such as methyldopa, doxazosin, clonidine and prazosin; angiotensin II antagonists such as irbesartan, candesartan cilextil, olmesartan medoxomil, valsartan, losartan, telmisartan and eprosartan mesylate; alpha/beta blockers such as carvedilol and labetalol; and diuretics such as bendroflumethiazide, piretanide, chlorthalidone and hydrochlorothiazide (HCTZ).

Advantages of the invention include the following:
- Riboflavin may be used to reduce blood pressure in the absence of any other antihypertensive agents.
- When other anti-hypertensive agents are administered with riboflavin, either simultaneously, separately or sequentially, the amount of the other antihypertensive agents necessary to have the required effect may advantageously be reduced, in that lesser amounts of the agents may be required in order to effect the treatment or prophylaxis of elevated blood pressure in a subject, thereby minimising any undesirable side effects of conventional anti-hypertensive agents.

According to a third aspect of the present invention, there is provided a product comprising riboflavin for use in the treatment or prophylaxis of elevated blood pressure in a subject homozygous or heterozygous for the MTHFR C677T polymorphism,

The medicament or product of the invention is preferably in a form suitable for oral or parenteral administration. Suitable oral dosage forms include tablets, capsules (including slow release capsules), pills, powders, granules and the like. Parenteral administration includes, for example, intravenous, intramuscular, intraarterial, intraperitoneal, intranasal, intravesical (e. g., to the bladder), intradermal, topical or subcutaneous administration. Suitable parenteral dosage forms include sterile injectable aqueous solutions or dispersions and sterile powders for the preparation of sterile injectable solutions or dispersions. The preferred route of administration is oral.

Riboflavin may be administered together with a pharmaceutically compatible or acceptable carrier suitable for oral or parenteral administration, selected according to the particular type of administration used. For oral administration, riboflavin may be administered with one or more solid inactive ingredients for the preparation of suitable oral dosage forms. For example, riboflavin may be administered with at least one excipient such as fillers, binders, humectants, disintegrating agents, solution retarders, absorption accelerators, wetting agents, absorbents or lubricating agents. For parenteral administration, riboflavin may be administered with a suitable carrier or diluent such as water, ethanol, saline solution, aqueous dextrose (glucose) and related sugar solutions, glycerol, or a glycol such as propylene glycol or polyethylene glycol.

In all cases, the dose of riboflavin will be within dietary reference levels up to and including the maximum level considered to be safe. Although, unlike many other nutrients, no official upper tolerable level has been established for riboflavin, it is generally considered to be safe even at very high doses with evidence showing that levels up to 500mg/day are tolerated with no adverse effects. This compares with recommended dietary levels of riboflavin for adults which range from 1.1 to 1.8mg/day. The medicament or product is most preferably formulated for administration of riboflavin to a subject in an amount of approximately 1.6 mg/day. However, the medicament or product may be formulated for administration of riboflavin to a subject in any other suitable dose greater or less than 1.6 mg/day. For example, a suitable dose may be from about 0.5 mg/day to 50 mg/day, preferably from about 0.75 mg/day to 20 mg/day, more preferably from about 1 mg/day to 10 mg/day, more preferably from about 1.2 mg/day to 5 mg/day, even more preferably from about 1.4 mg/day to about 5 mg/day.

The invention has application in the area of personalised nutrition. There is an increasing interest in gene-nutrient interactions, and with the greater accessibility of genetic profiling, the market for personalised nutrition is emerging in recent years. This approach is based on the view that dietary requirements should consider not only general factors such as age and sex, but also genetic factors which are specific to the subject. For example, a subject could check their MTHFR genotype and then proceed to take low-dose riboflavin, specifically if they are found to have the TT or CT genotype.

The invention also has application in the design of clinical drug trials, e.g. those testing new anti-hypertensive medication drugs. For the successful outcome and correct interpretation of such trials, it will be critical that those with the TT genotype for the MTHFR C677T polymorphism in combination with low riboflavin status are stratified and randomised equally to the different treatment groups.

### EXAMPLE 1: CVD PATIENTS

### MATERIALS AND METHODS

### Subject recruitment

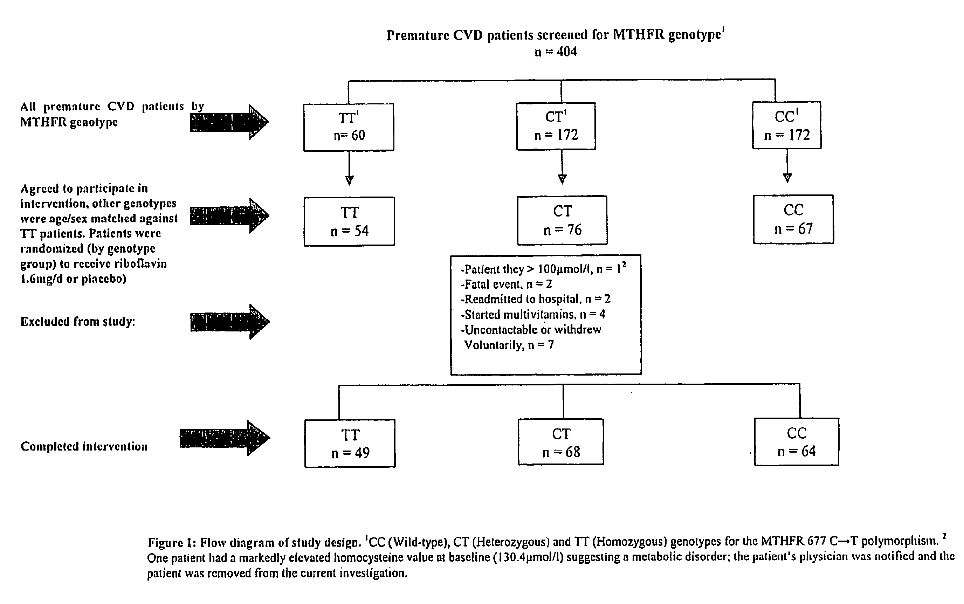

Ethical approval was granted by both the Research Ethical Committee of the University of Ulster and Altnagelvin Hospital Trust, Londonderry, Northern Ireland. Premature CVD patients, males aged 55 years and females aged 65 years and under at time of event were recruited from the cardiac department of Altnagelvin hospital. Patients were indicated by a previous myocardial infarction (MI) or angina diagnosed by ECG changes. Potential participants (n = 404) were screened for MTHFR genotype on the basis of a buccal swab sample. Those with the TT genotype were identified (n = 54); these were age and sex matched with a similar number of patients with the CC or CT genotypes, resulting in a total of 197 patients who were invited to participate in the current intervention study (see Figure 1). Exclusion criteria for all subjects were history of hepatic or renal disease, consumers of B-vitamin supplements, those taking medication known to interfere with folate metabolism, at least 3 months post MI prior to blood sampling. All volunteers provided written informed consent and completed a health and medical/lifestyle questionnaire which included questions regarding family history of CVD and smoking habits.

### Study design

The study was a 16-week placebo controlled intervention trial. Patients were stratified on the basis of their initial screening plasma homocysteine within each genotype group and subsequently randomised within each stratum to receive either riboflavin (1.6mg/day) or placebo, as indicated in Figure 1. In an attempt to maximise compliance, patients were provided with riboflavin every 4 weeks in 7-day pill boxes and asked to return any unused pills which were recorded. Non-fasting blood samples were collected at screening and post intervention either at Altnagelvin hospital, their workplace or their own homes.

### Blood pressure and anthropometric measurements

Blood pressure measurements were recorded as the mean of two separate measurements taken 15 minutes apart using an Omcron 705CP electronic blood pressure monitor (Medisave, Dorset, UK). Weight (kg) and height in (m), were used to calculate BMI (weight (kg)/height² (m)). Waist circumference (cm) was also measured. All measurements were made using Seca approved equipment (Brosch Direct, Ltd, Peterborough, UK).

### Sample collection

One 30ml blood sample was collected from each patient, one 9ml EDTA tube for plasma and washed red cells, one 4ml EDTA tube for the preparation of red blood cell lysates and the measurement of haemoglobin (HB) and packed cell volume (PCV); one 8ml serum separation tube for serum extraction, one 5ml serum separation tube for lipid profile analysis and one 4ml sodium citrate tube for coagulation screening. The 9ml EDTA was immediately wrapped in tin foil and placed on ice. Samples were centrifuged within 2 hours of sampling at 3000rpm for 15 minutes. Plasma was removed and stored at -80° C for plasma homocysteine and PLP analysis. The buffy layer was removed for confirmation of MTHFR genotyping and stored at -20°C. The remaining red blood cells were thrice washed with phosphate buffered saline (PBS) with the supernatant and remaining buffy layer being discarded after each wash. The washed red cells were removed and stored at - 80°C for EGRac analysis (a functional indicator of riboflavin status). Serum was removed and stored at -80°C for serum folate.

### Biochemical analysis

Plasma homocysteine was measured by immunoassay using the Abbott Imx analyser (Leino A., 1999, "Fully automated measurement of total homocysteine in plasma and serum on the Abbott IMx analyzer." Clin. Chem, 45, 569-71). Serum folate and red blood cell folate concentrations were determined by microbiological assay using the cryopreserved, microtitre plate method (Molloy et al, 1997, "Microbiological assay for serum, plasma, and red cell folate using cryopreserved, microtiter plate method." Methods Enzymol, 281, 43-53). Plasma PLP was determined by reverse-phase high pressure liquid chromatography (HPLC) with fluorescence detection (Bates et al, 1999, "Plasma pyridoxal phosphate and pyridoxic acid and their relationship to plasma homocysteine in a representative sample of British men and women aged 65 years and over." British Journal of Nutrition, 1, 191-201). Identification of the MTHFR C677T genotype was carried out using the polymerase chain reaction (PCR) amplification followed by HinFl (Frosst et al, 1995, "A candidate genetic risk factor for vascular disease: A common mutation in methylenetetrahydrofolate reductase." Nat Genetc, 10, 111-113). Riboflavin status was determined on the basis of EGRac, a functional assay whereby the activity of glutathione reductase is measured both with and without added FAD. EGRac is then calculated as a ratio of FAD-stimulated to unstimulated enzyme activity (Powers et al, 1983, "The relative effectiveness of iron and iron with riboflavin in correcting a microcytic anaemia in men and children in rural Gambia." Hum Nutr Clin Nutr, 37, 413-425), and values ≥1.3 are generally considered to reflect sub-optimal riboflavin status. Coagulation screening was measured by ACI Instrumentation Laboratories. HB and PCV were measured using Sysmex XE-2100 (Sysmex, UK Ltd. Milton Keynes UK) analyser and lipid profiles were measured using Abbott Architect CI 8200 analyser (Abbott Laboratories, USA). For all assays, samples were analysed blind, in duplicate and within 1 year of collection. Quality control was provided by repeated analysis of stored batches of pooled washed red blood cells (for EGRac), plasma for homocysteine and PLP and serum for folate covering a wide range of values.

### Statistical Analysis

All statistical analysis was performed using the SPSS statistical package for the social sciences (version 11.0, SPSS UK Ltd., Chersey, United Kingdom). For normalisation purposes variables were log-transformed as appropriate prior to statistical analysis. One way ANOVA with Tukey's post hoc test was used to examine the differences in the baseline characteristics among the genotype groups. Categorical data were assessed using chi squared analysis. Correlation analysis was performed using bivariate Pearson correlation coefficients. The effect of riboflavin intervention within each genotype group was examined using paired t-tests. P values < 0.05 were considered significant.

### RESULTS

### Description of Figures:

Figure 1 shows the flow diagram of study design.

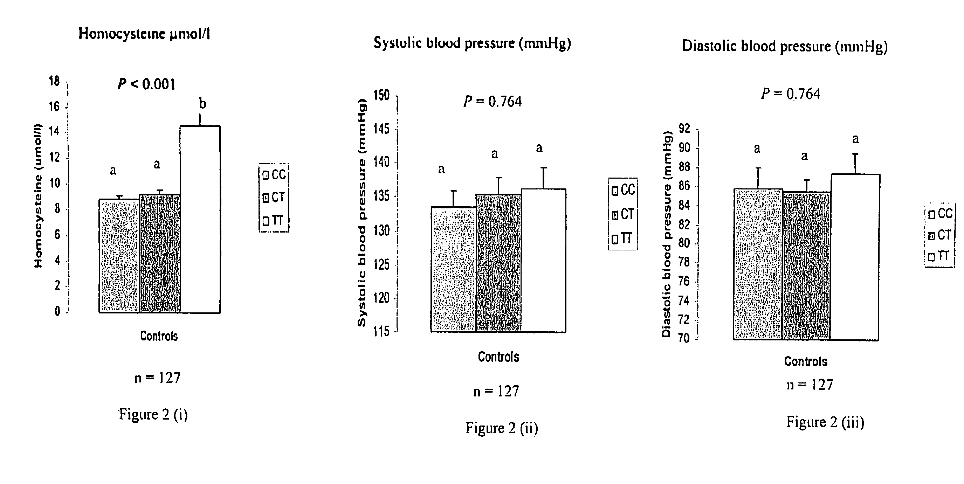

Figure 2(i) shows homocysteine concentration (µmol/l) by MTHFR genotype group among healthy controls; Figure 2(ii) shows systolic blood pressure (mmHg) by MTHFR genotype group among healthy controls; and Figure 2(iii) shows diastolic blood pressure (mmHg) by MTHFR genotype group among healthy controls. Values in Figures 2(i), 2(ii) and 2(iii) are mean (standard error bars). Different letters denote statistically significant differences between the MTHFR genotype groups, ANOVA with Tukey post-hoc test, P < 0.05 significant. In Figures 2(i)-2(iii), the letter "a" denotes values that do not differ significantly from other "a" values; and the letter "b" denotes values which do not differ significantly from other "b" values, but which do differ from the values denoted as "a".

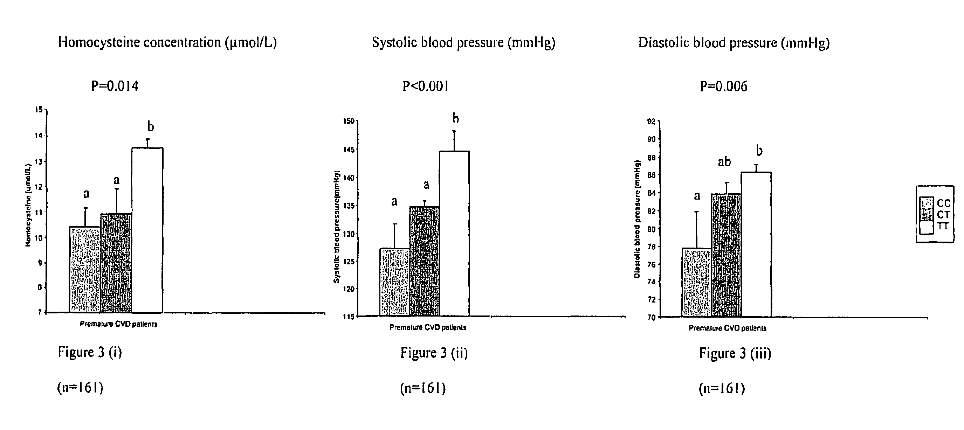

Figure 3(i) shows homocysteine concentration (µmol/l) by MTHFR genotype group among premature CVD patients; Figure 3(ii) shows systolic blood pressure (mmHg) by MTHFR genotype group among premature CVD patients; and Figure 3(iii) shows diastolic blood pressure (mmHg) by MTHFR genotype group among premature CVD patients. Values in Figures 3(i), 3(ii) and 3(iii) are mean (standard error bars). Different letters denote statistically significant differences between the MTHFR genotype groups, ANOVA with Tukey post-hoc test, P < 0.05 significant. In Figures 3(i)-3(iii), the letter "a" denotes values that do not differ significantly from other "a" values; and the letter "b" denotes values which do not differ significantly from other "b" values, but which do differ from the values denoted as "a". The letters "ab" denote values that do not differ significantly from a value denoted as "a" or "b".

### Baseline data in healthy individuals:

Referring to Figure 2, in relation to blood homocysteine levels, the phenotype observed for healthy individuals homozygous for MTHFR C677T polymorphism (i.e. TT genotype) was elevated blood homocysteine concentration, compared to those with either CT or CC genotypes. In relation to corresponding blood pressure levels, it was found that in healthy individuals with the TT genotype there was no elevation in the systolic or diastolic blood pressure compared to values in either CT or CC genotypes, as shown in Figure 2.

### Baseline data in patients with premature CVD:

Patients with premature CVD are generally defined as subjects who develop CVD before the age of 55 for men, or before the age of 65 for women. Referring to Figure 3, patients with premature CVD and with the TT genotype had elevated blood homocysteine concentration, compared with both CC and CT patients. Of note, TT patients were also found to have significantly increased systolic and diastolic blood pressure, as shown in Figure 3, compared to CC patients. CT individuals were found to have intermediate levels of diastolic blood pressure, compared to CC and TT patients (as shown in Figure 3). These results are unexpected, as healthy TT subjects showed no elevation in blood pressure compared with the other genotype groups. All patients were taking one or more antihypertensive drugs at the time of sampling, as indicated in Table 1 below, but it is clear from Figure 3 that the medication was relatively ineffective in the TT genotype group.

**Table 1**

| Anti-hypertensive medications taken by patients (n=161) at time of sampling | |
|---|---|
| **Anti-hypertensive medication** | **% of patients** |
| Betablockers | 38 |
| Ace Inhibitors | 18 |
| Calcium channel blockers | 6 |
| Diuretics | 3 |
| Ace inhibitors + Betablockers | 18 |
| Ace inhibitors + Diuretics | 4 |
| Ace inhibitors + Calcium channel blockers | 1 |
| Betablockers + Calcium channel blockers | 2 |
| Betablockers + Diuretics | 6 |
| Calcium channel blockers + Diuretics | 0.6 |
| Ace inhibitors + Calcium channel blockers + Diuretics | 0.6 |
| Betablockers + Calcium channel blockers + Diuretics | 0.6 |

The baseline characteristics of premature CVD patients sorted in accordance with their MTHFR genotype (TT, CT, or CC) are shown in Table 2 below. Referring to Table 2, it was observed that TT patients had significantly elevated systolic and diastolic blood pressure, compared to CC patients. CT patients were found to have intermediate values for diastolic blood pressure. Of note, the elevated blood pressure levels found in TT patients were strongly influenced by riboflavin status; patients with the combination of the TT genotype and low riboflavin status were unexpectedly found to have markedly elevated blood pressure.

**Table 2 Baseline characteristics of premature cardiovascular disease patients split by MTHFR 677C→T genotype**

| | *MTHFR genotype* | | | | |
|---|---|---|---|---|---|
| | All | CC | CT | TT | P |
| General characteristics | (n = 197) | (n = 67) | (n = 76) | (n = 54) | |
| Age now (y) | 53.2 (5.8) | 53.4 (6.1) | 52.6 (5.0) | 54.0 (6.4) | 0.382 |
| Age at time of event (y) | 46.9 (6.1) | 47.1 (5.6) | 46.9(5.4) | 47.1(7.5) | 0.891 |
| Male (%) | 75.1 | 78.1 | 69.7 | 78.2 | 0.303 |
| Family history of CVD (%) | 68.0 | 68.7 | 65.8 | 70.4 | 0.763 |
| Family history of premature CVD (%) | 41.6 | 40.3 | 50.0 | 31.5 | 0.127 |
| Current smoker (%) | 32.0 | 31.3 | 27.6 | 38.9 | 0.209 |
| Body mass index (kg/m2) | 29.1 (4.6) | 29.3 (4.8) | 28.7 (4.5) | 29.3 (4.5) | 0.724 |
| Waist circumference (cm) | 95.1 (12.1) | 96.3(11.9) | 94.2 (11.4) | 95.0 (13.4) | 0.625 |
| Prothrombin time (sec) | 13.9 (3.1) | 13.7 (2.8) | 14.1 (3.4) | 13.6 (2.3) | 0.700 |
| Fibrinogen concentration (g/l) | 3.93 (0.92) | 3.93 (0.84) | 3.97 (1.05) | 3.88 (0.83) | 0.853 |
| Total cholesterol (mmol/l) | 4.5 (0.9) | 4.4 (0.81) | 4.5 (0.8) | 4.5 (1.0) | 0.849 |
| **B-vitamin status** | | | | | |
| Plasma homocysteine (µmol/l) | 10.9(5.3) | 9.8 (3.3)^{a} | 10.5 (3.9)^{ab} | 12.8 (8.0)^{b} | 0.007 |
| EGRac | 1.38 (0.20) | 1.37 (0.17) | 1.39 (0.22) | 1.37 (0.19) | 0.854 |
| Red cell folate (nmol/l) | 959(455) | 1030(518)^{a} | 1011 (400)^{a} | 796(408)^{b} | 0.009 |
| Pyridoxal phosphate (nmol/) (B-6) | 61.0(37.0) | 68.0(35.0)^{a} | 63.2(40.4)^{ab} | 49.4(32.0)^{b} | 0.017 |
| | | | | | |
| **Blood pressure** | | | | | |
| Systolic Blood Pressure (mmHg) | 135.0 (19.6) | 131.1 (18.0)^{a} | 133.0 (19.7)^{a} | 142.8 (19.5)^{b} | 0.002 |
| *By riboflavin status:* | | | | | |
| Lower riboflavin status | 137.1 (20.5) | 131.2 (20.4)^{a} | 135.8 (19.0)^{a} | 147.4 (19.8)^{b} | 0.005 |
| Higher riboflavin status | 132.5 (18.5) | 131.0 (14.8) | 129.6 (20.4) | 138.6 (19.2) | 0.172 |
| | | | | | |
| Diastolic Blood Pressure(mmHg) | 83.0 (12.2) | 80.3 (12.5)^{a} | 83.3 (11.5)^{ab} | 86.0(12.3)^{b} | 0.038 |
| *By riboflavine status:* | | | | | |
| Lower riboflavin status | 84.1 (12.8) | 80.8 (13.5) | 84.6(11.8) | 88.1 (12.7) | 0.076 |
| Higher riboflavin status | 81.7(11.4) | 79.6(11.2) | 81.9(11.1) | 84.1 (12.2) | 0.381 |

| | | | | | |
|---|---|---|---|---|---|
| Values are presented as mean (SD). Values among genotypes groups were compared using one way ANOVA with tukey post hoc tests. Abbreviations: MTHFR, methylenetetrahydrofolate reductase; erythrocyte glutathione reductase activation coefficient (riboflavin status, a higher EGRac value indicates lower vitamin status); 'Higher' and 'lower' riboflavin status was determined using the median EGRac value within each genotype group as a cut-off. | | | | | |

### Intervention data in patients with premature CVD

Referring to Table 3 below, in a placebo controlled intervention study in 181 patients with premature CVD, significant lowering of both systolic and diastolic blood pressure in patients with the TT genotype was achieved following 16 weeks of low-dose riboflavin administration. The blood pressure response shown is of both statistical and clinical significance. The magnitude of blood pressure lowering (a decrease of 11 units in systolic and 8 in diastolic blood pressure) observed in response to riboflavin can be estimated from meta-analyses to lower the risk of heart disease by 29% and stroke by 46%. The results show that a significant and clinically important reduction in blood pressure was achieved in response to riboflavin, specifically in patients with the TT genotype. Referring again to Table 3, no significant reduction was observed in blood pressure (either systolic or diastolic) in CC patients following riboflavin administration.

**Table 3. Response to riboflavin intervention for 16 weeks in premature CVD patients.**

| | **MTHFR Genotype** | | | | | |
|---|---|---|---|---|---|---|
| | **CC Placebo (n = 32)** | | | **CC Riboflavin (n = 32)** | | |
| | **Pre** | **Post** | ***P*** | **Pre** | **Post** | ***P*** |
| Homocysteine(µmol/l) | 9.5(2.8) | 9.3(2.9) | 0.474 | 10.1 (3.9) | 9.8(3.6) | 0.271 |
| EGRac | 1.40(0.19) | 1.42(0.20) | 0.520 | 1.34(0.16) | 1.24(0.07) | <0.001 |
| Serum folate(µg/l) | 12.1(8.5) | 14.0(9.6) | 0.052 | 10.5(6.5) | 12.0(7.5) | 0.094 |
| Red cell folate(nmol/l) | 1063(589) | 1139(561) | 0.245 | 1021(557) | 1075(526) | 0.427 |
| Systolic blood pressure (mmHg) | 126.6(20.4) | 129.0(22.1) | 0.421 | 134.3(14.9) | 133.4(15.3) | 0.733 |
| Diastolic blood pressure (mmHg) | 79.3(14.9) | 80.9(15.5) | 0.546 | 80.1(8.9) | 80.4(12.0) | 0.881 |

| | **CT Placebo (n=33)** | | **CT Riboflavin (n=35)** | | | |
|---|---|---|---|---|---|---|
| | **Pre** | **Post** | ***P*** | **Pre** | **Post** | ***P*** |
| Homocysteine(µmol/l) | 11.2(4.1) | 10.8(4.6) | 0.327 | 10.5(3.8) | 10.6(6.3) | 0.881 |
| EGRac | 1.41(0.22) | 1.44 (0.29) | 0.209 | 1.38(0.24) | 1.25(0.13) | <0.001 |
| Serum folate(µg/l) | 7.83(4.34) | 8.12(6.44) | 0.744 | 10.41(6.61) | 11.80(11.01) | 0.342 |
| Red cell folate (nmol/l) | 991(388) | 991(434) | 0.997 | 1008(400) | 1022(460) | 0.759 |
| Systolic blood pressure (mmHg) | 133.6(21.5) | 129.9(21.5) | 0.241 | 136.2(16.9) | 135.4(15.4) | 0.708 |
| Diastolic blood pressure (mmHg) | 82.0(12.1) | 81.9(14.2) | 0.962 | 86.0(10.8) | 84.2(9.2) | 0.336 |

| | **TT Placebo (n = 24)** | | **TT Riboflavin (n = 25)** | | | |
|---|---|---|---|---|---|---|
| | **Pre** | **Post** | ***P*** | **Pre** | **Post** | ***P*** |
| Homocysteine(µmol/l) | 11.8(6.7) | 11.8(7.8) | 0.915 | 11.3(4.4) | 10.0(3.1) | 0.014 |
| EGRac | 1.32(0.12) | 1.34(0.11) | 0.298 | 1.41(0.20) | 1.27(0.09) | <0.001 |
| Serum folate(µg/l) | 7.7(6.6) | 8.9(7.5) | 0.157 | 8.1(4.8) | 9.6(8.1) | 0.244 |
| Red cell folate (nmol/1) | 808(488) | 903(580) | 0.164 | 845(303) | 889(306) | 0.305 |
| Systolic blood pressure (mmHg) | 143.8(18.1) | 141.6(22.5) | 0.566 | 142.8(22.1) | 131.6(20.9) | 0.005 |
| Diastolic blood pressure (mmHg) | 84.5(10.7) | 85.4(12.7) | 0.739 | 88.1(14.2) | 80.2(14.2) | 0.002 |

| | | | | | | |
|---|---|---|---|---|---|---|
| Values are presented as mean (SD). Pre and post intervention values within each treatment group were compared using paired t-test. Abbreviations: MTHFR, methylenetetrahydrofolate reductase; EGRac, erythrocyte glutathione reductase activation coefficient (riboflavin status, higher values indicate lower status). | | | | | | |

### EXAMPLE 2: HEALTHY CONTROLS

The association between riboflavin and blood pressure in healthy individuals according to MTHFR C677T genotype was investigated. Measurements were taken and recorded according to the procedures described in Example 1 above. The results obtained, namely the baseline characteristics of healthy subjects pre-screened and age-matched for MTHFR genotype (n=124), are shown in Table 4.

**Table 4**

| | ***MTHFR* C677T Genotype** | | | |
|---|---|---|---|---|
| **General characteristics** | **CC** (n =46) | **CT** (n =34) | **TT** (n = 44) | ***P*** |
| Age (y) | 51.4(4.0) | 51.4(3.0) | 50.8(5.1) | 0.718 |
| Male (%) | 69.6 | 85.3 | 75 | 0.264 |
| BMI (kg/m²) | 28.1 (4.8) | 28.0 (4.2) | 28.6 (4.6) | 0.791 |
| Waist circumference (cm) | 90.4 (11.9) | 90.6 (12.3) | 93.6 (13.5) | 0.434 |
| Systolic BP(mmHg) | 132.9 (16.5) | 134.8 (15.8) | 137.4 (19.8) | 0.473 |
| Diastolic BP(mmHg) | 84.1 (13.2) | 86.4 (8.7) | 88.1 (13.9) | 0.315 |
| | | | | |
| **Biochemical measurements** | | | | |
| Plasma homocysteine (µmol/l) | 9.3 (1.9)a | 8.9 (2.1)a | 14.1 (8.0)b | <0.001 |
| Riboflavin status (EGRac) | 1.34 (0.17) | 1.37 (0.15) | 1.38 (0.16) | 0.492 |
| Red cell folate (nmol/l) | 874 (357) | 955 (433) | 748 (412) | 0.071 |
| Vitamin B6 status (PLP; nmol/l) | 79.1 (47.4) | 79.7 (38.7) | 68.3 (35.5) | 0.370 |

| | | | | |
|---|---|---|---|---|
| Values are mean (SD). ANOVA was used to compare values among the genotype groups; an overall P value < 0.05 indicates a statistically significant difference among the 3 genotype groups. The letters show where the differences are occurring: different letters (a,b) indicate significant differences between any two genotypes, whereas the same letter indicates that values were not significantly different between any two genotypes (Tukey post hoc tests). Thus, the letter "a" denotes values that do not differ significantly from other "a" values; and the letter "b" denotes values which do not differ significantly from other "b" values, but which do differ from the values denoted as "a". | | | | |

As shown by the results obtained in Example 2 (Table 4), individuals with the TT genotype did not show significantly higher systolic or diastolic blood pressure, compared with the CC or CT genotype groups. However, they did demonstrate the typical phenotype for this TT genotype of significantly elevated plasma homocysteine and a tendency towards lower folate status, though, as shown in Table 4, the difference in folate status did not reach statistical significance (since the P value was not < 0.05).

### Intervention data in healthy individuals

A placebo controlled intervention study in 81 healthy individuals was carried out. Blood pressure response to riboflavin intervention (1.6mg/day for 16 weeks) by MTHFR genotype in healthy individuals was investigated, and the results are shown in Table 5.

Referring to the P values, the results of Table 5 show that there was no significant systolic or diastolic blood pressure lowering effect of riboflavin in this healthy cohort with the TT genotype. Therefore, the lowering of systolic and diastolic blood pressure by riboflavin in CVD patients having the TT genotype discussed above in Example 1 was unexpected, and is of statistical and clinical significance.

Referring again to the P values shown in Table 5, the statistical analysis shows that there are no significant increases or decreases in blood pressure in response to riboflavin or placebo across the genotypes, with one exception, namely that diastolic blood pressure did show an increase with riboflavin treatment in the CC group. Whilst this is unexpected, this result may be attributed to chance and the relatively small sample size. Even taking this response to riboflavin in the CC group into account, it is noted that riboflavin was found to increase the diastolic blood pressure in healthy CC individuals, with the result that the lowering of systolic and diastolic blood pressure by riboflavin in CVD patients having the TT genotype discussed above in Example 1 is particularly unexpected.

**Table 5. Blood Pressure response to riboflavin intervention (1.6mg/d, 16weeks) by MTHFR genotype in healthy subjects (n=81)**

| **MTHFR 677C → T Genotype** | | | | | | | | | | |
|---|---|---|---|---|---|---|---|---|---|---|
| | | **CC (n=25)** | | **CT (n=30)** | | | **TT (n=26)** | | | |
| | | **Before** | **After** | **p-value** | **Before** | **After** | **p-value** | **Before** | **After** | **p-value** |
| **Systolic blood pressure (mmHg)** | | | | | | | | | | |
| Placebo | median | 136.0(124.7, 140.2) | 129.0(120.3, 141.0) | 0.663 | 147.0(136.5, 151.5) | 142.0(132.1, 156.1) | 0.981 | 146.0(135.5, 157.8) | 150.0(141.6, 157.8) | 0.395 |
| Riboflavin treatment | median | 137.0(121.9, 148.4) | 143.5(129.8, 156.2) | 0.077 | 150.0(135.5, 153.3) | 150.0(137.2, 153.4) | 0.797 | 139.5(129.9, 142.5) | 145.0(133.3, 151.1) | 0.970 |

| **Diastolic blood pressure (mmHg)** | | | | | | | | | | |
|---|---|---|---|---|---|---|---|---|---|---|
| Placebo | median | 83.0(77.2, 93.0) | 83.0(76.6, 85.74) | 0.286 | 87.0(83.7, 92.8) | 88.0(81.3, 95.0) | 0.970 | 90.0(82.8, 94.1) | 86.0(83.6, 95.5) | 0.698 |
| Riboflavin treatment | median | 80.5(73.7, 88.3) | 86.5(78.7, 91.9) | 0.033 | 87.0(82.7, 94.2) | 89.0(83.3, 94.7) | 0.767 | 88.5(81.1, 91,6) | 88.5(82.8, 95.1) | 0.172 |

| | | | | | | | | | | |
|---|---|---|---|---|---|---|---|---|---|---|
| Values are median (95% confidence interval). Before and After intervention blood pressures were compared using a paired t-test. A P value < 0.05 indicates a statistically significant difference before versus after intervention. | | | | | | | | | | |

## Claims

1. Use of riboflavin in the manufacture of a medicament for the treatment or prophylaxis of elevated blood pressure in a subject homozygous for the MTHFR C677T polymorphism.

2. Use of riboflavin in the manufacture of a medicament for the treatment or prophylaxis of elevated blood pressure in a subject heterozygous for the MTHFR C677T polymorphism.

3. Use of riboflavin according to claim 1 or claim 2, wherein the medicament is in a form suitable for oral or parenteral administration.

4. Use of riboflavin according to any one of claims 1-3, wherein the riboflavin is administered with a pharmaceutically compatible or acceptable carrier.

5. A pharmaceutical product for use in the treatment or prophylaxis of elevated blood pressure in a subject homozygous for the MTHFR C677T polymorphism, comprising a pharmaceutically effective amount of an anti-hypertensive agent and riboflavin, the anti-hypertensive agent and the riboflavin being for simultaneous, separate or sequential administration.

6. A pharmaceutical product for use In the treatment or prophylaxis of elevated blood pressure in a subject heterozygous for the MTHFR C677T polymorphism, comprising riboflavin and a pharmaceutically effective amount of an anti-hypertensive agent, the riboflavin and the antihypertensive agent being for simultaneous, separate or sequential administration.

7. A pharmaceutical product for use according to claim 5 or claim 6, wherein the antihypertensive agent is selected from ACE inhibitors, beta blockers, Ca⁺⁺ antagonists, alpha blockers, angiotensin II antagonists, alpha/beta blockers, and diuretics.

8. A pharmaceutical product according to claim 7, wherein the ACE inhibitors are selected from quinapril, captopril, lisinopril, benazepril, perindopril, enalapril maleate, trandolapril, ramipril and cilazapril; the beta blockers are selected from atenolol, tertatolol, metoprolol tartrate, bisoprolol fumarate, nebbivolol, celiprolol and pindolol; the Ca⁺⁺ antagonists are selected from nifedipine, diltiazem, amiodipine, verapamil and felopidine; the alpha blockers are selected from methyldopa, doxazosin, clonidine and prazosin; the anglotensin II antagonists are selected from irbesartan, candesartan cilextil, olmesartan medoxomil, valsartan, losartan, telmisartan and eprosartan mesylate; the alpha/beta blockers are selected from carvedllol and tabetalol; and the diuretics are selected from bendroflumethiazide, piretanide, chlorthalidone and hydrochlorothiazide (HCTZ).

9. A pharmaceutical product according to any one of claims 5-8, wherein the pharmaceutical product is in a form suitable for oral or parenteral administration.

10. Product comprising riboflavin for use in the treatment or prophylaxis of elevated blood pressure in a subject homozygous for the MTHFR C677T polymorphism.

11. Product comprising riboflavin for use in the treatment or prophylaxis of elevated blood pressure in a subject heterozygous for the MTHFR C677T polymorphism,

12. Product comprising riboflavine for use according to claim 10 or 11, the product comprising a nutrition product.

13. Product comprising riboflavin for use according to claim 10 or 11, the product comprising a product for personalized nutrition.

## Patentansprüche

1. Verwendung von Riboflavin bei der Herstellung eines Medikaments für die Behandlung oder Prophylaxe von erhöhtem Blutdruck bei einem Subjekt, das für den MTHFR C677T Polymorphismus homozygot ist.

2. Verwendung von Riboflavin bei der Herstellung eines Medikaments für die Behandlung oder Prophylaxe von erhöhtem Blutdruck bei einem Subjekt, das für den MTHFR C677T Polymorphismus heterozygot ist.

3. Verwendung von Riboflavin nach Anspruch 1 oder Anspruch 2, worin das Medikament in einer Form vorliegt, die zur oralen oder parenteralen Verabreichung geeignet ist.

4. Verwendung von Riboflavin nach einem der Ansprüche 1-3, worin das Riboflavin mit einem pharmazeutisch kompatiblen oder verträglichen Träger verabreicht wird.

5. Pharmazeutisches Produkt zur Verwendung bei der Behandlung oder Prophylaxe von erhöhtem Blutdruck bei einem Subjekt, das für den MTHFR C677T Polymorphismus homozygot ist, das eine pharmazeutisch wirksame Menge eines blutdrucksenkenden Mittels und Riboflavin umfasst, wobei das blutdrucksenkende Mittel und das Riboflavin zur gleichzeitigen, separaten oder sequentiellen Verabreichung vorgesehen sind.

6. Pharmazeutisches Produkt zur Verwendung bei der Behandlung oder Prophylaxe von erhöhtem Blutdruck bei einem Subjekt, das für den MTHFR C677T Polymorphismus heterozygot ist, das Riboflavin und eine pharmazeutisch wirksame Menge eines blutdrucksenkenden Mittels umfasst, wobei das Riboflavin und das blutdrucksenkende Mittel zur gleichzeitigen, separaten oder sequentiellen Verabreichung vorgesehen sind.

7. Pharmazeutisches Produkt zur Verwendung nach Anspruch 5 oder Anspruch 6, worin das blutdrucksenkende Mittel aus Folgenden ausgewählt ist: ACE-Hemmern, Beta-Blockern, Ca⁺⁺-Antagonisten, Alpha-Blockern, Angiotensin-II-Antagonisten, Alpha/Beta-Blockern und Diuretika.

8. Pharmazeutisches Produkt nach Anspruch 7, worin die ACE-Hemmer aus Folgenden ausgewählt sind: Quinapril, Captopril, Lisinopril, Benazepril, Perindopril, Enalaprilmaleat, Trandolapril, Ramipril und Cilazapril; die Beta-Blocker aus Folgenden ausgewählt sind: Atenolol, Tertatolol, Metoprololtartrat, Bisoprololfumerat, Nebivolol, Celiprolol und Pindolol; die Ca⁺⁺-Antagonisten aus Folgenden ausgewählt sind: Nifedipin, Diltiazem, Amlodipin, Verapamil und Felodipin; die Alpha-Blocker aus Folgenden ausgewählt sind: Methyldopa, Doxazosin, Clonidin und Prazosin; die Angiotensin-II-Antagonisten aus Folgenden ausgewählt sind: Irbesartan, Candesartan cilextil, Olmesartan medoxomil, Valsartan, Losartan, Telmisartan und Eprosartan-Mesylat; die AlphaBeta-Blocker aus Folgenden ausgewählt sind: Carvedilol und Labetalol; und die Diuretika aus Folgenden ausgewählt sind: Bendroflumethiazid, Piretanid, Chlorthalidon und Hydrochlorothiazid (HCTZ).

9. Pharmazeutisches Produkt nach einem der Ansprüche 5-8, worin das pharmazeutische Produkt in einer Form vorliegt, die zur oralen oder parenteralen Verabreichung geeignet ist.

10. Produkt, das Riboflavin umfasst, zur Verwendung bei der Behandlung oder Prophylaxe von erhöhtem Blutdruck bei einem Subjekt, das für den MTHFR C677T Polymorphismus homozygot ist.

11. Produkt, das Riboflavin umfasst, zur Verwendung bei der Behandlung oder Prophylaxe von erhöhtem Blutdruck bei einem Subjekt, das für den MTHFR C677T Polymorphismus heterozygot ist.

12. Produkt, das Riboflavin umfasst, zur Verwendung nach Anspruch 10 oder 11, wobei das Produkt ein Ernährungsprodukt umfasst.

13. Produkt, das Riboflavin umfasst, zur Verwendung nach Anspruch 10 oder 11, wobei das Produkt ein Produkt für die personalisierte Ernährung umfasst.

## Revendications

1. Utilisation de la riboflavine dans la fabrication d'un médicament pour le traitement ou la prophylaxie de la pression artérielle élevée chez un sujet homozygote pour le polymorphisme C677T du gène MTHFR.

2. Utilisation de la riboflavine dans la fabrication d'un médicament pour le traitement ou la prophylaxie de la pression artérielle élevée chez un sujet hétérozygote pour le polymorphisme C677T du gène MTHFR.

3. Utilisation de la riboflavine selon la revendication 1 ou la revendication 2, où le médicament est sous une forme appropriée à une administration orale ou parentérale.

4. Utilisation de la riboflavine selon l'une quelconque des revendications 1-3, où la riboflavine est administrée avec un véhicule pharmaceutiquement compatible ou acceptable.

5. Produit pharmaceutique conçu pour être utilisé dans le traitement ou la prophylaxie de la pression artérielle élevée chez un sujet homozygote pour le polymorphisme C677T du gène MTHFR, qui comprend une quantité pharmaceutiquement efficace d'un agent antihypertensif et de riboflavine, l'agent antihypertensif et la riboflavine étant administrés simultanément, séparément ou en succession.

6. Produit pharmaceutique conçu pour être utilisé dans le traitement ou la prophylaxie de la pression artérielle élevée chez un sujet hétérozygote pour le polymorphisme C677T du gène MTHFR, qui comprend une quantité pharmaceutiquement efficace d'un agent antihypertensif et de riboflavine, l'agent antihypertensif et la riboflavine étant administrés simultanément, séparément ou en succession.

7. Produit pharmaceutique conçu pour une utilisation selon la revendication 5 ou la revendication 6, où l'agent antihypertensif est sélectionné parmi des inhibiteurs de l'ECA, des bêta-bloquants, des antagonistes du Ca⁺⁺, des alpha-bloquants, des antagonistes de l'angiotensine II, des alpha/bêta-bloquants et des diurétiques.

8. Produit pharmaceutique selon la revendication 7, où les inhibiteurs de l'ECA sont sélectionnés parmi le quinapril, le captopril, le lisinopril, le bénazépril, le périndopril, le maléate d'énalapril, le trandolapril, le ramipril et le cilazapril ; les bêta-bloquants sont sélectionnés parmi l'aténolol, le tertatolol, le tartrate de métoprolol, le fumarate de bisoprolol, le nébivolol, le céliprolol et le pindolol ; les antagonistes du Ca⁺⁺ sont sélectionnés parmi la nifédipine, le diltiazem, l'amlodipine, le vérapamil et la félodipine ; les alpha-bloquants sont sélectionnés parmi la méthyldopa, la doxazosine, la clonidine et la prazosine ; les antagonistes de l'angiotensine II sont sélectionnés parmi l'irbésartan, le candésartan cilexétil, l'olmésartan médoxomil, le valsartan, le losartan, le telmisartan et le mésylate d'éprosartan ; les alpha/bêta-bloquants sont sélectionnés entre le carvédilol et le labétalol ; et les diurétiques sont sélectionnés parmi le bendrofluméthiazide, le pirétanide, la chlorthalidone et l'hydrochlorothiazide (HCTZ).

9. Produit pharmaceutique selon l'une quelconque des revendications 5-8, où le produit pharmaceutique est sous une forme appropriée à une administration orale ou parentérale.

10. Produit comprenant la riboflavine conçu pour une utilisation dans le traitement ou la prophylaxie de la pression artérielle élevée chez un sujet homozygote pour le polymorphisme C677T du gène MTHFR.

11. Produit comprenant la riboflavine conçu pour une utilisation dans le traitement ou la prophylaxie de la pression artérielle élevée chez un sujet hétérozygote pour le polymorphisme C677T du gène MTHFR.

12. Produit comprenant la riboflavine conçu pour une utilisation selon la revendication 10 ou 11, le produit comprenant un produit de nutrition.

13. Produit comprenant la riboflavine conçu pour une utilisation selon la revendication 10 ou 11, le produit comprenant un produit pour nutrition personnalisée.
